# EUROPEAN PATENT APPLICATION

(11) **EP 4 183 361 A1**
(43) Date of publication of application: **24.05.2023**
(21) Application number: 22208951.8
(22) Date of filing: 22.11.2022
(51) Int. Cl.: A61B 18/14, A61B 1/00

(54) **ABLATION DEVICE FOR ATTACHMENT TO AN ENDOSCOPE**

(30) Priority: 22.11.2021 US 202163281965 P
(71) Applicant: Cook Medical Technologies LLC, Bloomington, IN 47404 (US)
(72) Inventor: MCCARTHY, Dillion, Bearnafunshin (IE)
(74) Representative: Leach, Sean Adam

(57) **Abstract**

An ablation cap including a body having a lumen for receiving a distal end of an endoscope, the body having a central axis extending therethrough, and at least one guide for receiving at least one lateral extension of an electrode platform, wherein at least a portion of the at least one guide extends at an angle relative to the central axis of the body.

## Description

### RELATED APPLICATION

This application claims the benefit of U.S. Provisional Application No. 63/281,965, filed on November 22, 2021, pending, the entirety of which is herein incorporated by reference.

### TECHNICAL FIELD

The present disclosure generally relates to ablation devices for attachment to an endoscope, and more particularly, to ablation devices, with a pivoting electrode, for attachment to an endoscope.

### BACKGROUND

Endoscopic devices and procedures may be used to diagnose, monitor and treat various conditions by close examination of the internal organs. By way of background, a conventional endoscope generally is an instrument having an imaging device for visualizing the interior of an internal region of a body and a lumen for inserting one or more treatment devices therethrough. A wide range of applications have been developed for the general field of endoscopes including by way of non-limiting example the following: arthroscope, angioscope, bronchoscope, choledochoscope, colonoscope, cytoscope, duodenoscope, enteroscope, esophagogastro-duodenoscope (gastroscope), laparoscope, laryngoscope, nasopharyngo-neproscope, sigmoidoscope, thoracoscope, and utererscope (individually and collectively, "endoscope").

By way of non-limiting example, millions of people suffer from progressive gastroesophageal reflux disease (GERD), which is characterized by frequent episodes of heartburn, typically on at least a daily basis. Without adequate treatment, GERD can cause erosion of the esophageal lining as the lower esophageal sphincter (LES), a segment of smooth muscle located at the junction of the stomach and the esophagus, gradually loses its ability to function as the barrier that prevents stomach acid reflux. Chronic GERD can also cause metaplasia to the inner lining of the esophagus where the normal squamous mucosa changes to columnar mucosa, also known as Barrett's esophagus. Barrett's esophagus can progress to esophageal cancer if left untreated.

Endoscopic treatment of Barrett's esophagus includes endoscopic mucosal resection (EMR). One method of performing EMR involves ablation of the mucosal surface by heating the surface until the surface layer is no longer viable. The dead tissue is then removed. Treatment devices for performing EMR have been developed using bipolar ablation technology that includes attaching an ablation cap to the distal end of an endoscope, then positioning a probe associated with the cap against the target tissue and delivering energy to the tissue to ablate the tissue in contact with the probe. In some devices, as a safety precaution, if the probe does not make sufficient contact with tissue to be ablated, the probe may not be energized.

Treatment with such devices is not limited to the esophagus. Rather, target sites for ablation may include tissue within the stomach, lower colon, small intestine, and rectum, among other locations. Such tissues may not be substantially flat, and may be formed just inside the entry to an organ. Due to anatomical geometry at such locations, and/or the tortous path leading to target sites in such locations, rigid electrode platforms and drive catheters may prevent a probe from making the desired contact with the tissue to be ablated, thereby preventing optimal treatment. Moreover, if adequate contact with tissue is not obtainable, such probe may not be energized.

By way of example, a target tissue site formed in the wall of the stomach may be curved, whether concave or convex, and may be located just beyond the cardia, for example, in a region of the fundus. A rigid electrode platform and drive catheter entering the stomach through the esophagus may not be positionable to reach the target tissue and/or make the desired contact for purposes of ablation. By way of further example, a target tissue site formed in the colon or small intestine may be formed on or just beyond a sharp bend in those organs, making delivery and sufficient contact of a probe with a rigid electrode platform and drive catheter particularly challenging, if not impossible.

What is needed in the art is an ablation treatment device that is simple to use, that is coupled to the endoscope, that minimizes the number of steps and time required for a treatment procedure, and that provides improved treatment of target tissue sites formed in challenging locations.

### SUMMARY

The present embodiments provide systems and methods suitable for ablation treatment using an endoscope, while i) maintaining suitable visibility of the target treatment site and surrounding environment, and ii) providing increased flexibility and maneuverability of an ablation device.

In one aspect, an ablation cap includes a body having a lumen for receiving a distal end of an endoscope, the body having a central axis extending therethrough, and, at least one guide for receiving at least one lateral extension of an electrode platform, wherein at least a portion of the at least one guide extends at an angle relative to the central axis of the body.

In some embodiments, the angle relative to the central axis of the body may be between 30 degrees and 60 degrees. Alternatively, the angle relative to the central axis of the body may be less than 30 degrees.

In some embodiments, the at least one guide may include a proximal portion parallel to the central axis of the body. In some embodiments, the at least one guide may include a distal portion angled relative to the central axis of the body.

In some embodiments, the ablation cap may further include a cover portion extending from a side of the body, the cover portion defining a recess between the cover portion and the body. The at least one guide may extend along at least a portion of the cover portion within the recess.

In some embodiments, the body may include an angled portion formed at an angle relative to the central axis of the body. The at least one guide may extend along the angled portion.

In some embodiments, the at least one guide includes a first portion extending at a first angle relative to the central axis of the body and a second portion extending at a second angle relative to the central axis of the body, the second angle being different than the first angle.

In some embodiments, the at least one guide is a channel. Alternatively, the at least one guide may be a rail.

In some embodiments, the at least one guide may include a first guide and a second guide, the first guide and the second guide being parallel.

In some embodiments, the body may be tubular.

The ablation device may include any one or more of the features above.

In another embodiment, an ablation device includes a body having a lumen for receiving a distal end of an endoscope, the body having a central axis extending therethrough, a cover portion extending from a side of the body, the cover portion defining a recess between the cover portion and the body, and an electrode platform having at least one lateral extension, the electrode platform movable between a covered position, where the electrode platform is covered by the cover portion, and an exposed position, where the electrode platform is not covered by the cover portion. At least one guide receives the at least one lateral extension of the electrode platform, wherein a portion of the at least one guide extends at an angle relative to the central axis of the tubular body.

In some embodiments, the at least one extension of the electrode platform is slidable within the at least one guide. The at least one lateral extension may include a hook for engaging the at least one guide. Alternatively, the at least one lateral extension may include a wheel for engaging the at least one guide. Or, the at least one lateral extension may include a mechanical bearing for engaging the at least one guide.

In some embodiment, the ablation device includes at least one electrode formed on the electrode platform. The ablation device may further include a drive catheter extending proximally from the electrode platform. The electrode platform may be pivotable relative to the drive catheter.

In some embodiments, the angle relative to the central axis of the body may be between 30 degrees and 60 degrees. Or, the angle relative to the central axis of the body may be less than 30 degrees.

In some embodiments, the at least one guide may include a proximal portion parallel to the central axis of the body. The at least one guide may include a distal portion angled relative to the central axis of the body. The at least one guide may extend along at least a portion of the cover portion within the recess.

In some embodiments, the body includes an angled portion formed at an angle relative to the central axis of the body. The at least one guide may extend along the angled portion.

In some embodiments, the at least one guide includes a first portion extending at a first angle relative to the central axis of the body and a second portion extending at a second angle relative to the central axis of the body, the second angle being different than the first angle.

In some embodiments, the at least one guide may be a channel. Alternatively, the at least one guide may be a rail. The at least one guide may include a first guide and a second guide, the first guide and the second guide being parallel.

In some embodiments, the body may tubular.

The ablation device may include any one or more of the features listed above.

In another embodiment, a user interface of an ablation device includes a slot having a proximal end and a distal end, a trigger disposed in the slot, the trigger at least partially extending through the slot, and being movable between the proximal end and the distal end, and a drive catheter operatively coupled to the trigger, the drive catheter extending distally from the user interface and operatively coupled to an electrode platform. The slot may have a first portion and a second portion distal of the first portion, wherein when the trigger is in the first portion, the electrode platform is in a first orientation, and, wherein when the trigger is in the second portion, the electrode platform is in a second orientation, the second orientation being angled relative to the first orientation.

In some embodiments, the first portion and the second portion may be separated by a resistance mechanism configured to resist the movement of the trigger from the first portion to the second portion.

In some embodiments, the user interface further includes a third portion proximal the first portion, wherein the third portion and the first portion are separated by a resistance mechanism configured to resist the movement of the trigger from the third portion to the first portion.

The user interface may have any one or more of the features listed above. Additionally, the user interface may be used with any of the ablation devices described above.

In yet another embodiment, an ablation device system includes an endoscope having an imaging device for capturing images at a distal end of the endoscope, one or more lumens extending through at least a portion of the endoscope between a proximal end of the endoscope and the distal end, and, an ablation device according to any of the ablation devices described above. The ablation device system may further include a user interface as described above.

Other systems, methods, features and advantages of the described embodiments will be, or will become, apparent to one with skill in the art upon examination of the following figures and detailed description. It is intended that all such additional systems, methods, features and advantages be within the scope of the disclosure, and be encompassed by the following claims.

### BRIEF DESCRIPTION OF THE DRAWINGS

The embodiments can be better understood with reference to the following drawings and description. The components in the figures are not necessarily to scale, emphasis instead being placed upon illustrating the principles of the disclosure. Moreover, in the figures, like referenced numerals designate corresponding parts throughout the different views.
FIG. 1 is a perspective view of an ablation cap with an electrode platform in a covered position, in accordance with an embodiment of the present disclosure;
FIG. 2 is a perspective view of the ablation cap shown in FIG. 1, with the electrode platform in an extended position;
FIG. 3 is a side view of the ablation cap shown in FIG. 1;
FIG. 4 is an front view the ablation cap shown in FIG. 1;
FIG. 5 is a rear view of the ablation cap shown in FIG. 1;
FIG. 6 is a cross-sectional side view of the ablation cap shown in FIG. 1, with the electrode platform in a covered position, taken along line 6-6 in Figure 4.
FIG. 7 is a partial view of an embodiment of a support member of the ablation cap;
FIG. 8 is a partial view of an embodiment of a support member of the ablation cap
FIG. 9 illustrates an embodiment of an electrode of the ablation cap;
FIG. 10 illustrates an embodiment of an electrode of the ablation cap;
FIG. 11 illustrates the distal end of an exemplary endoscope for use with the ablation caps of the present disclosure;
FIG. 12 is a perspective view of an ablation cap, electrode platform, and drive catheter, in accordance with another embodiment of the present disclosure, illustrating the electrode platform in a partially extended position;
FIG. 13 is a perspective view of the ablation cap, electrode platform, and drive catheter shown in FIG. 12, illustrating the electrode platform in a fully extended, pivoted position;
FIG. 14 is a side view of the ablation cap, electrode platform, and drive catheter shown in FIG. 12, illustrating the electrode platform in a retracted position, comparable to that shown in FIG. 1;
FIG. 15 is a cross-sectional side view of the ablation cap, electrode platform, and drive catheter shown in FIG. 12, with the electrode platform in a retracted position;
FIG. 16 is a cross-sectional side view of the ablation cap, electrode platform, and drive catheter shown in FIG. 12, with the electrode platform in a partially extended position, comparable to that shown in FIG. 12;
FIG. 17 is a cross-sectional side view of the ablation cap, electrode platform, and drive catheter shown in FIG. 12, with the electrode platform in a fully extended, pivoted position, comparable to that shown in FIG. 13; and,
FIGS. 18A-C are partial top views of a user interface for moving the electrode platform and drive catheter of FIG. 12 between the positions illustrated in FIGS. 12-17.

### DETAILED DESCRIPTION

In the present application, the term "proximal" refers to a direction that is generally towards a physician during a medical procedure, while the term "distal" refers to a direction that is generally towards a target site within a patient's anatomy during a medical procedure. As used herein to describe example embodiments, the term "fluid" may refer to a gas or a liquid.

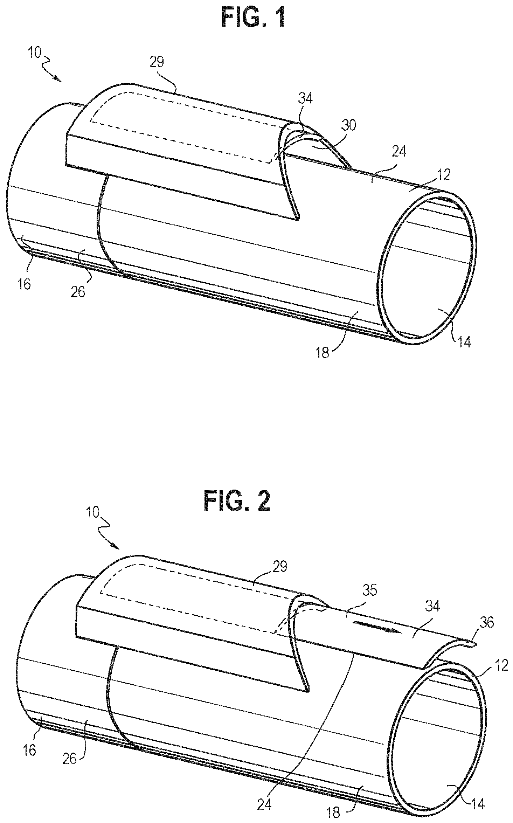

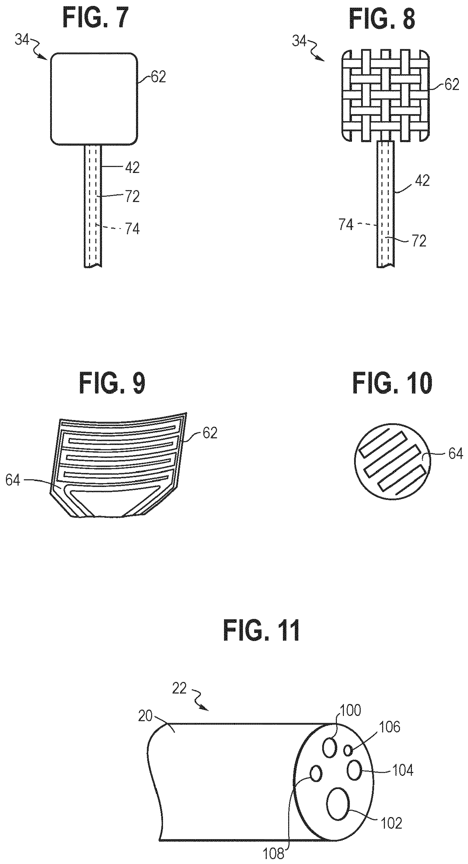

FIGS. 1-6 illustrate an embodiment of an ablation cap 10 in accordance with the present disclosure. As shown, the ablation cap 10 includes a tubular body 12 having a lumen 14 formed therein. The ablation cap 10 includes a proximal portion 16 and a distal portion 18. The proximal portion 16 of the cap 10 is sized to fit on a distal end 20 of an endoscope 22 (shown in FIG. 11). In some embodiments, the proximal portion 16 of the ablation cap 10 may include a flexible portion 26 that is connected to the tubular body 12 and that fits over the distal end 20 of the endoscope 22 to secure the cap 10 to the endoscope 22, for example, by friction fit. In some embodiments, the proximal portion 16 may be made of a hard material that is sized and shaped to fit over the distal end 20 of the endoscope 22 by friction fit.

The distal portion 18 of the ablation cap 10 may extend beyond the distal end 20 of the endoscope 22. The distal portion 18 may be cylindrical. In some embodiments, the distal portion 18 may be formed from a material having sufficient transparency so that the operator using an imaging device 100 of the endoscope 22 may observe a portion of the tissue to be treated by viewing the tissue through a wall 24 of the distal portion 18 of the ablation cap 10. The distal portion 18 may also include a portion that is formed from a material for magnifying the tissue under observation.

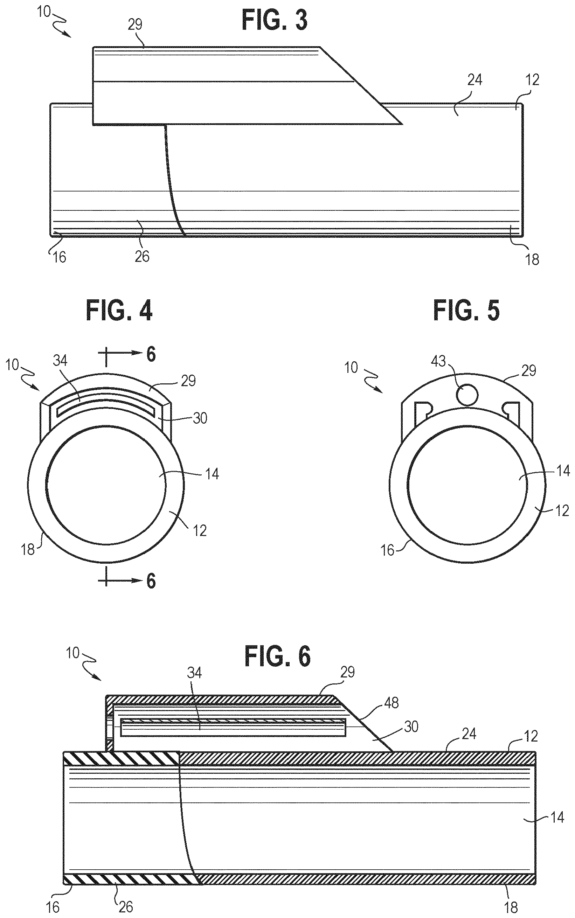

The cap 10 may further include a hood or a cover portion 29 that includes a recess 30 formed as part of the ablation cap 10. The cover portion 29 may be integrally formed with the cap 10 or provided as a separate portion and connected to the cap 10. The cover portion 29 is at least partially spaced apart from the tubular body to form the recess 30. The recess 30 may be sized and shaped to hold an extendable electrode platform 34 within the recess 30 in a covered position, as shown in FIGS. 1 and 6. The electrode platform 34 is slidably positionable within the recess 30 of the cover portion 29. In some embodiments, the electrode platform 34 may be positioned entirely within the recess 30 of the cover portion 29 in the covered position so that electrodes positioned on the electrode platform 34 are completely covered. As shown in FIG. 2, the electrode platform 34 may be extended distally from the recess 30 so that at least a portion of a surface 35 of the electrode platform is exposed and can contact the tissue to be treated. A portion of the wall 24 is positioned behind the electrode platform 34 when the electrode platform 34 is an exposed position and may be used to support the electrode platform 34 when the electrode platform 34 is pressed against the tissue to be treated. In some embodiments, a distal end 36 of the electrode platform 34 does not extend beyond a distal end of the distal portion 18 of the cap 10.

In some embodiments, a distal end 36 of the electrode platform 34 is extended less than the extension as shown in FIG. 2. By way of non-limiting example, the distal end 36 may be extended less than 100% or about 20%, 40%, 60% and 80% of the extension shown in FIG. 2. Other extension distances are also possible. In some embodiments, the electrode platform 34 may be colored to facilitate viewing the electrode platform 34 as it is advanced distally and to determine the amount that the electrode platform 34 has been extended. For example, the electrode platform 34 may be black or blue or any color that may be seen through an endoscope to help viewing the position of the electrode platform 34. In some embodiments, the cap 10 may include a stop to stop the electrode platform 34 at a maximum extension and to prevent the electrode platform from extending too far out of the cap 10.

In some embodiments, at least a portion of the electrode platform 34 may be viewable through the endoscope. The electrode platform 34 may move into and out of the view of the endoscope, for example, when the electrode platform 34 has been extended a certain percent relative to the cap 10, the electrode platform 34 may be viewed through the endoscope. By way of non-limiting example, the electrode platform 34 may be viewed when 10%, 20%, 30%, 40%, 50%, 60%, 70%, 80%, 90%, 100% or other amount has been extended distally from the retracted position of FIG. 1. Electrodes positioned on the electrode platform 34 may also be energized when the electrode platform 34 is extended distally less than 100%.

A cross-sectional side view of the ablation cap 10 is shown in FIG. 6. The lumen 14 extends through the ablation cap 10 between the proximal portion 16 and the distal portion 18. The electrode platform 34 is shown within the recess 30 of the cover portion 29. In the embodiment shown, a beveled portion 48 is positioned on a distal edge of the recess 30. The beveled portion 48 may be used to help prevent tissue entrapment within the recess 30, for example, by scraping ablated tissue off the electrode platform 34, when the electrode platform 34 is retracted in a proximal direction, to a position within the cover portion 29.

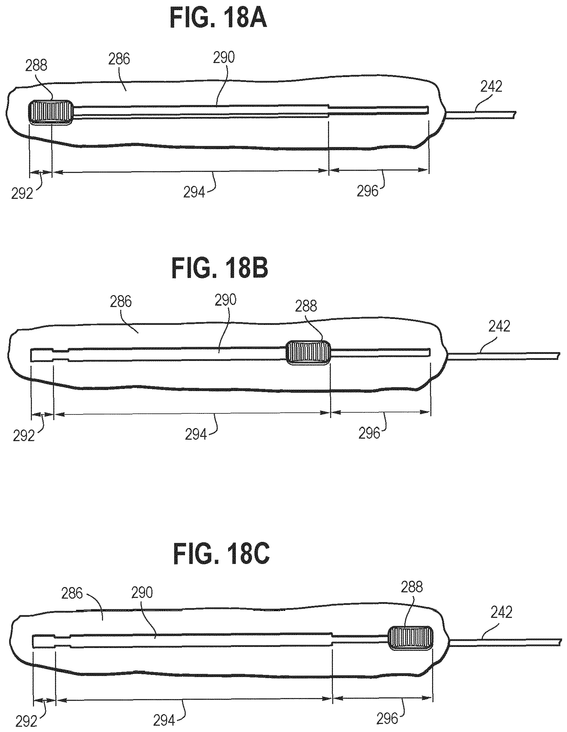

As shown in FIGS. 7 and 8, the electrode platform 34 may be connected to a drive catheter 42 that extends proximally from the electrode platform 34, through an opening 43 (see FIG. 5) in the rear of the cover portion 39, to a proximal control handle or user interface 286 (see FIGS. 18A-C). The drive catheter 42 is distally movable to extend the electrode platform 34 from the recess 30 of the cover portion 29 and proximally movable to re-position the electrode platform 34 within the recess 30. Typically, the electrode platform 34 is positioned within the recess 30 of the cover portion 29 when the ablation cap 10 is being delivered to a treatment site or being repositioned within a patient's lumen for additional treatment at one or more additional sites. Positioning of the electrode platform 34 within the recess 30 also helps to prevent accidental energy delivery, for example to healthy tissue. The electrode platform 34 is at least partially distally extended from the recess 30 of the cover portion 29 for treatment at a site and energy is delivered to the tissue to ablate the diseased tissue as described in more detail below.

In some embodiments, the electrode platform 34 may include a support member 62 upon which one or more electrodes 64 are positioned. FIGS. 7 and 8 illustrate exemplary support members 62. As shown in FIG. 7, the support member 62 may be a solid material, such as a plastic material. As shown in FIG. 8, the support member 62, may be a mesh. When the solid material or the mesh is formed of a metallic material, a layer of insulation may be provided between the support member 62 and the electrodes 64. The support member 62 may be moved proximally and distally with the drive catheter 42. The electrodes 64 may be secured to the support member 62 by any method known to one skilled in the art. By way of non-limiting example, the electrodes may be secured by gluing, bonding, taping, an adhesive backing on the electrodes, crimping, manufacturing the electrodes directly on to the body and the like.

Electrical wires 72 may extend through a lumen 74 of the drive catheter 42 as shown in FIGS. 7 and 8 and connect to the electrodes 64 to supply the energy for ablation. Alternatively, the electrical wires 72 may extend through a lumen of the endoscope 22. Exemplary electrodes 64 may be seen in FIGS. 8 and 9. The electrodes 64 may be provided separately from the support member 62 and in some embodiments may also form the support member 62 without providing a separate support member.

As shown in FIGS. 8 and 9, the electrodes 64 may include positive electrodes 64 and negative electrodes 64 in a bipolar device. When provided as a bipolar device, the electrodes 64 are provided in pairs, one positive and one negative electrode per pair. The electrodes 64 may also be provided as a monopolar device having a single electrode 64 or a plurality of electrodes 64 with a grounding pad or an impedance circuit additionally provided (not shown). The electrodes 64 may be provided in any pattern on the support member 62. The electrodes 64 may cover the entire support member 64 or a portion thereof. By way of non-limiting example, a space 62 between the positive electrode 64 and the negative electrode 64 may between about 0.1 mm to about 5 mm. In some embodiments, the energy may be delivered to the tissue for a period of time from about 0.1 second to about 10 seconds. In some embodiments, the amount of energy delivered to the tissue may be from about 10 watts to about 60 watts. Other spacing distances between electrodes, length of time, and energy delivery are also possible and depend on the target tissue, the depth of the lesion, the type of energy, the length of application of the energy to the tissue and the spacing of the electrodes.

The electrodes 64 are operably connected to an energy source (not shown). In some embodiments, the energy source may be a radio frequency source. However, other types of energy sources may also be used to provide energy to the electrodes. By way of non-limiting example, additional possible energy sources may include microwave, ultraviolet, cryogenic and laser energies.

In some embodiments, the ablation cap may be made primarily of a substantially transparent or translucent polymer such as polytetrafluorothylene (PTFE). Additional possible materials include, but are not limited to the following, polyethylene ether ketone (PEEK), fluorinated ethylene propylene (FEP), perfluoroalkoxy polymer resin (PFA), polyamide, polyurethane, high density or low density polyethylene, and nylon. In some embodiments, the ablation cap may be formed from a lubricious material such as PTFE and the like for easy slidability within the patient's lumen for delivery to the treatment site. In some embodiments, the ablation cap or a portion thereof may be formed from magnifying or other image enhancing materials. The ablation cap or a portion thereof may also be coated or impregnated with other compounds and materials to achieve the desired properties. Exemplary coatings or additives include, but are not limited to, parylene, glass fillers, silicone hydrogel polymers and hydrophilic coatings.

FIG. 11 illustrates the distal end 20 of an exemplary endoscope 22 for use with the ablation caps of the present disclosure. In some embodiments, the endoscope 22 may include an imaging device 100 extending through at least a portion of endoscope 22 between a proximal end of the endoscope 22 and the distal end 20. The imaging device 100 may include a lens operatively coupled to, e.g., in signal communication with, an external imaging system and is configured to capture images of the target site and transmit signals indicative of the captured images to the imaging system. The endoscope 22 may also include an accessory channel 102 extending through at least a portion of the endoscope 20 between a proximal end of the endoscope 22 and the distal end 20. The accessory channel 102 may be used to delivery any number of accessories or devices, such as a catheter (not shown), to the distal end 22 of the endoscope 20. Endoscope 22 may further include one or more light sources 104, such as light-emitting diodes (LEDs), and a water source 106 configured to inject water into the target site. Finally, the endoscope 22 may include one or more fluid lumens 108 extending through at least a portion of the endoscope 20 between a proximal end of the endoscope 22 and the distal end 20. The one or more fluid lumens 108 may be in fluid communication with an external source of a pressurized fluid (e.g., gas such as carbon dioxide or a liquid) to aerate the target site. Alternatively, or additionally, the one or more fluid lumens 108 may be in fluid communication with a vacuum pump to withdraw fluid from the target site and/or to supply a suction force within the cap 10 in order to draw the tissue to be ablated into contact with the electrodes 64.

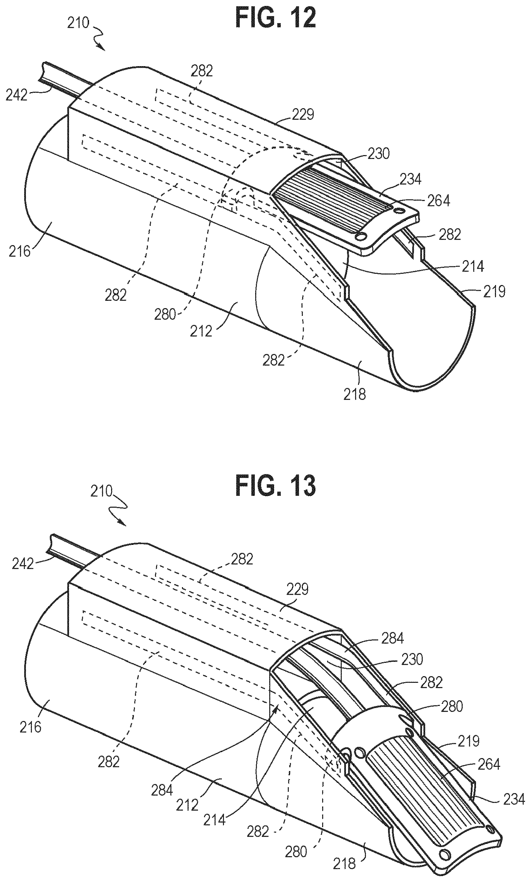

FIGS. 12-17 illustrate another embodiment of an ablation cap, electrode platform, and drive catheter in accordance with the present disclosure. The ablation cap, electrode platform, and drive catheter of FIGS. 12-17 may be used in conjunction with the endoscope 22 of FIG. 11, or other endoscopes. The structure of the ablation cap, electrode platform, and drive catheter of FIGS. 1-11 is exemplary of the structures of the ablation cap, electrode platform, and drive catheter illustrated in FIGS. 12-17, except as described below.

Similar to prior embodiments, FIGS. 12-17 illustrate an ablation cap 210 having a tubular body 212 having a lumen 214 formed therein. The ablation cap 210 includes a proximal portion 216 and a distal portion 218. The proximal portion 216 of the cap 210 is sized to fit on a distal end 20 of an endoscope 22 (shown in FIG. 11). In some embodiments, the proximal portion 216 of the ablation cap 210 may include a flexible portion that is connected to the tubular body 212 and that fits over the distal end 20 of the endoscope 22 to secure the cap 210 to the endoscope 22, for example, by friction fit. In some embodiments, the proximal portion 216 may be made of a hard material that is sized and shaped to fit over the distal end 20 of the endoscope 22 by friction fit.

The distal portion 218 of the ablation cap 210 may extend beyond the distal end 20 of the endoscope 22. The distal portion 218 may form a generally cylindrical wall. However, unlike the ablation cap 10, in the distal portion 218 of the ablation cap 210, the tubular body 212 forms an angled portion 219, providing for movement of an electrode platform in a direction of or toward the angled portion 219. In some embodiments, the distal portion 218 may be formed from a material having sufficient transparency so that the operator using an imaging device 100 of the endoscope 22 may observe a portion of nearby tissue to be treated by viewing the tissue through a wall of the distal portion 218 of the ablation cap 210. The distal portion 218 may also include a portion that is formed from a material for magnifying the tissue under observation.

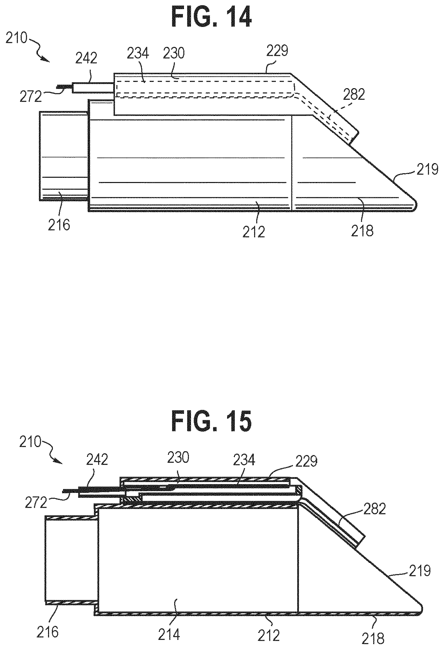

The cap 210 may further include a hood or a cover portion 229 that includes a recess 230 formed as part of the ablation cap 210. The cover portion 229 may be integrally formed with the cap 210 or provided as a separate portion and connected to the cap 210. The cover portion 229 is at least partially spaced apart from the tubular body to form the recess 230. The recess 230 may be sized and shaped to hold an extendable electrode platform 234 within the recess 230 in a covered position, as shown in FIGS. 14-15, and comparable to the position illustrated in FIG. 1. The electrode platform 234 is slidably positionable within the recess 230 of the cover portion 229. In some embodiments, the electrode platform 234 may be positioned entirely within the recess 230 of the cover portion 229 in the covered position so that electrodes positioned on the electrode platform 234 are completely covered. As shown in FIG. 12, the electrode platform 234 may be extended distally from the recess 230 so that at least a portion of a surface of the electrode platform is exposed and can contact the tissue to be treated. In some embodiments, the angled portion 219 is positioned below the electrode platform 234 when the electrode platform 234 is an exposed position and may be used to support the electrode platform 234 when the electrode platform 234 is pressed against the tissue to be treated.

In some embodiments, at least a portion of the electrode platform 234 may be viewable through the endoscope. The electrode platform 234 may move into and out of the view of the endoscope, for example, when the electrode platform 234 has been extended a certain percent relative to the cap f210, the electrode platform 234 may be viewed through the endoscope. By way of non-limiting example, the electrode platform 234 may be viewed when 10%, 20%, 30%, 40%, 50%, 60%, 70%, 80%, 90%, 100% or other amount has been extended distally from the retracted position of FIG. 15. Electrodes positioned on the electrode platform 34 may also be energized when the electrode platform 34 is extended distally less than 100%.

As shown in FIGS. 12-17, the electrode platform 234 may be connected to a drive catheter 242 that extends proximally from the electrode platform 234, through an opening (not shown) in the rear of the cover portion 239, to a proximal control handle or user interface 286, described below. In some embodiments, the drive catheter 234 may be connected to the dive catheter via a mechanical hinge, in order to allow the electrode platform to rotate or pivot relative to the drive catheter. In other embodiments, the electrode catheter may be connected to the drive catheter 242 via a living hinge, for example, formed by a flexible material, or a reduction in material.

The drive catheter 242 is distally movable to extend the electrode platform 234 from the recess 230 of the cover portion 229 and proximally movable to re-position the electrode platform 234 within the recess 230. Typically, the electrode platform 234 is positioned within the recess 230 of the cover portion 229 when the ablation cap 210 is being delivered to a treatment site or being repositioned within a patient's lumen for additional treatment at one or more additional sites. Positioning of the electrode platform 234 within the recess 230 also helps to prevent accidental energy delivery, for example to healthy tissue. The electrode platform 234 is at least partially distally extended from the recess 230 of the cover portion 229 for treatment at a site, and energy is delivered to the tissue to ablate the diseased tissue as described in more detail below.

The electrode platform 234 may include a support member upon which one or more electrodes 264 are positioned. The electrode platform 234, and the one or more electrodes 264, may be constructed and formed in the same manner as the electrode platform 34, and the electrodes 64, described above. Electrical wires 272 may extend through a lumen of the drive catheter 242 and connect to the electrodes 264 to supply the energy for ablation. Alternatively, the electrical wires 272 may extend through a lumen of the endoscope 22 .

In some embodiments, as described below, the electrode platform may include one or more lateral extensions 280 configured to slidably engage one or more guides 282 formed on the ablation cap 210. The lateral extensions may be formed, for example, as wings (as shown), or as cylindrical rods. Notably, when the lateral extensions 280 are formed as cylindrical rods, the lateral extensions 280 and electrode platform 234 may rotate or pivot within the slot, about a central axis of the rods.

The ablation cap 210 differs from the ablation cap 10 in that it includes at least one guide 282 formed in the hood or cover portion 229, and at least along a portion of the angled portion 219 of the tubular body 212. In some embodiments, as shown, the ablation cap 210 includes two opposing guides 282. The guides 282 may be formed as a channel, or a slot, formed in the cover portion 229, and/or along the angled portion 219, and are configured to receive the lateral extensions 280 of the electrode platform 282 in a sliding engagement. Notably, because the lateral extensions 280 are received within the guides 282, the lateral extensions 280 are not exposed to tissue, thereby preventing an end or a portion of the lateral extensions 280 from catching onto any tissue, which could cause perforation of the tissue, when the electrode platform 234 and lateral extensions 280, pivot in and/or slide along the guides 282. In other embodiments, the guides 282 may be formed as a rail on which the lateral extensions are slidably mounted, for example, with a hook structure, a wheel, or other mechanical bearing.

The guides 282 may form multiple portions. For example, in a first portion formed within the hood or cover portion 229, the guides 282 may extend in the proximal-distal direction, generally parallel to a central axis of the tubular body 212. In a second portion formed along the angled portion 219, the guides 282 may extend at an angle relative to the central axis of the tubular body 212 and/or the direction of the guides 282 formed in the cover portion 229. In some embodiments, the guides 282 may transition from the first portion, extending in the proximal-distal direction, to the second portion, formed along the angled 219, via a curved portion 284. The curved portion may be configured to enable the lateral extensions 280 to slide within the guides 282 from the first portion to the second portion. In some embodiments, the guides 282 may extend along the angled portion 219 at an angle of 45 degrees relative to the central axis of the tubular body 212. In other embodiments, the guides 282 may extend along the angled portion 219 at an angle of 30 to 60 degrees relative to the central axis of the tubular body 212. In other embodiments, the guides 282 may extend along the angled portion 219 at an angle of less than 30 degrees relative to the central axis of the tubular body 212.

In this way, as the electrode platform 234 is advanced via the drive catheter 242 distally from a retracted position, shown in FIGS. 14-15, to an exposed position, as shown in FIG. 12, the lateral extensions 280 slide in the guides 282 along the first portion formed in the hood 229 to guide the electrode platform 234, and expose at least a portion of the one or more electrodes 264 formed on the electrode platform 234. The electrodes 264 may be selectively energized to ablate tissue when the electrode platform 234 is advanced to the general position shown in FIG. 12. Or, as the electrode platform 234 is further advanced via the drive catheter 242 distal of the position shown in FIG. 12, the lateral extensions 280 slide in the guides 282 through the curved portion 284 and into the second portion, formed along the angled portion 219 of the tubular body 212, as shown in FIG. 13, thereby guiding the electrode platform 234 (and electrodes 264 formed thereon) at an angle relative to the central axis of the tubular body 212. In effect, the guides 282 cause the electrode platform 234 to rotate relative to the central axis of the tubular body 212 to the angle of the second portion.

In yet other embodiments, the guides 282 may extend along the angled portion 219 at multiple different angles relative to the central axis of the tubular body 212. For example, the guides 282 may include a first portion formed within the hood or cover portion 229, a second portion extending along the angled portion 219 at an angle of 30 degrees relative to the central axis of the tubular body 212, and a third portion, distal of the second portion, extending along the angled portion 219 at an angle of 45 degrees relative to the central axis of the tubular body 212. In yet other embodiments, the guides 282 may extend along the angled portion 219 through a range of gradually increasing angles relative to the central axis of the tubular body 212 to form a curved portion, where advancing the lateral extensions 280 distally through the curved portion of the guides 282 gradually increases the angle of the electrode platform 234 relative to the central axis of the tubular body 212.

As described above, the ablation cap 210 and rotatable or pivotable electrode platform 234 provide a clinician with an ablation device having increased flexibility and maneuverability, thereby enabling a wider range of permissible treatment sites and applications, as compared to ablation devices having a rigid electrode platform and drive catheter. And, as with existing ablation devices, the ablation cap 210 may be attached to the distal end of an endoscope, permitting endoscopic visualization of the target tissue and ablation treatment, while providing the increased functionality described herein.

In some embodiments, the electrode platform 234 and drive catheter 242 may be coupled to a proximal control handle, or user interface. For example, FIGS. 18A-C are partial top views of a proximal control handle or user interface 286 for moving the electrode platform 234 and drive catheter 242 between the positions illustrated in FIGS. 12-17. The user interface 286 may include a button or trigger 288 slidably mounted within a slot 290 and operatively coupled to the drive catheter 242, such that movement of the trigger 288 results is movement of the drive catheter 242 (and electrode platform 234). The button or trigger 288 may extend from through the slot 290 for engagement with and movement by a finger of a user.

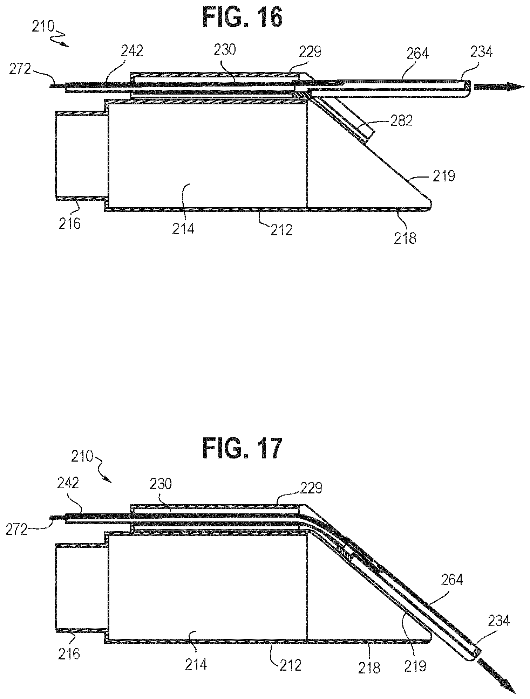

In some embodiments, the slot 290 may comprise a plurality of regions. For example, a first region 292 may be defined as a fully-retracted position, where the electrode platform 234 is fully retracted within the hood or cover portion 229 of the ablation cap 210, as illustrated in FIGS. 14-15, and comparable to the position shown in FIG. 1. A second region 294 may be defined as an extended region, where the electrode platform 234 is at least partially extended from the hood or cover portion 229 of the ablation cap 210, such that at least a portion of the electrodes 264 disposed thereon are exposed, and wherein the electrode platform 234 moves in the proximal-distal direction, without any rotation or pivoting, as illustrated in FIGS. 12 and 16. A third region 296 may be defined as a rotation or pivoting region, where entire the electrode platform 234 is extended beyond the hood or cover portion 229, such that the electrodes 264 are fully exposed, and wherein the electrode platform 234 moves in the proximal-distal direction, but with rotation or pivoting, as illustrated in FIGS. 13 and 17.

In some embodiments, the slot 290 may have one or more locking and/or resistance mechanisms disposed therein to provide a user with tactile feedback and/or resist movement of the trigger 288 within the slot 290. Suitable locking or resistance mechanisms may include protrusions, detents, frictional zones, narrowed slot width, or other suitable means. For example, a resistance mechanism may be associated with a proximal end of the first region 292, corresponding to the fully retracted position of the electrode platform 234, illustrated in FIGS. 14 and 15, such that movement of the trigger 288 from the first region 292 into the second region 294 requires the user to apply a threshold amount of force to the trigger 288 in the distal direction. Another resistance mechanism may be associated with a distal end of the second region 294, corresponding to a fully extended position, without rotation or pivoting of the electrode platform 234, illustrated in FIG. 16, such that further movement of the trigger 288 from the second region 294 into the third region 296 requires the user to apply a threshold amount of force to the trigger 288 in the distal direction. Movement of the trigger 288 to the distal end of the slot 290 in the third region 296 would then move and rotate or pivot the electrode platform 234 to the position illustrated in FIGS. 13 and 17, where the lateral extensions 280 of the electrode 234 have reached the distal end of the guides 282.

Although the subject matter has been described in language specific to structural features and/or methodological acts, it is to be understood that the subject matter defined in the appended claims is not necessarily limited to the specific features or acts described. Rather, the specific features and acts are disclosed as illustrative forms of implementing the claims.

One skilled in the art will realize that a virtually unlimited number of variations to the above descriptions are possible, and that the examples and the accompanying figures are merely to illustrate one or more examples of implementations.

It will be understood by those skilled in the art that various other modifications can be made, and equivalents can be substituted, without departing from claimed subject matter. Additionally, many modifications can be made to adapt a particular situation to the teachings of claimed subject matter without departing from the central concept described herein. Therefore, it is intended that claimed subject matter not be limited to the particular embodiments disclosed, but that such claimed subject matter can also include all embodiments falling within the scope of the appended claims, and equivalents thereof.

In the detailed description above, numerous specific details are set forth to provide a thorough understanding of claimed subject matter. However, it will be understood by those skilled in the art that claimed subject matter can be practiced without these specific details. In other instances, methods, devices, or systems that would be known by one of ordinary skill have not been described in detail so as not to obscure claimed subject matter.

The present disclosure comprises the following numbered clauses:
1. An ablation cap comprising:
   a body having a lumen for receiving a distal end of an endoscope, the body having a central axis extending therethrough; and,
   at least one guide for receiving at least one lateral extension of an electrode platform, wherein at least a portion of the at least one guide extends at an angle relative to the central axis of the body.
2. The ablation cap of clause 1, wherein the angle relative to the central axis of the body is between 30 degrees and 60 degrees.
3. The ablation cap of clause 1, wherein the angle relative to the central axis of the body is less than 30 degrees.
4. The ablation cap of any preceding clause, wherein the at least one guide comprises a proximal portion parallel to the central axis of the body.
5. The ablation cap of any preceding clause, wherein the at least one guide comprises a distal portion angled relative to the central axis of the body.
6. The ablation cap of any preceding clause, further comprising a cover portion extending from a side of the body, the cover portion defining a recess between the cover portion and the body.
7. The ablation cap of any preceding clause, wherein the at least one guide extends along at least a portion of the cover portion within the recess.
8. The ablation cap of any preceding clause, wherein the body comprises an angled portion formed at an angle relative to the central axis of the body.
9. The ablation cap of clause 8, wherein the at least one guide extends along the angled portion.
10. The ablation cap of any preceding clause, wherein the at least one guide includes a first portion extending at a first angle relative to the central axis of the body and a second portion extending at a second angle relative to the central axis of the body, the second angle being different than the first angle.
11. The ablation cap of any preceding clause, wherein the at least one guide is a channel.
12. The ablation cap of any preceding clause, wherein the at least one guide is a rail.
13. The ablation cap of any preceding clause, wherein the at least one guide comprises a first guide and a second guide, the first guide and the second guide being parallel.
14. The ablation cap of any preceding clause, wherein the body is tubular.
15. The ablation cap of clause 1 according to any one or more of clauses 2 to 14.
16. An ablation device comprising:
   a body having a lumen for receiving a distal end of an endoscope, the body having a central axis extending therethrough;
   a cover portion extending from a side of the body, the cover portion defining a recess between the cover portion and the body;
   an electrode platform having at least one lateral extension, the electrode platform movable between a covered position, where the electrode platform is covered by the cover portion, and an exposed position, where the electrode platform is not covered by the cover portion; and,
   at least one guide for receiving the at least one lateral extension of the electrode platform, wherein a portion of the at least one guide extends at an angle relative to the central axis of the tubular body.
17. The ablation device of clause 16, wherein the at least one extension of the electrode platform is slidable within the at least one guide.
18. The ablation device of clause 16 or 17, wherein the at least one lateral extension comprises a hook for engaging the at least one guide.
19. The ablation device of any of clauses 16 to 18, wherein the at least one lateral extension comprises a wheel for engaging the at least one guide.
20. The ablation device of any of clauses 16 to 19, wherein the at least one lateral extension comprises a mechanical bearing for engaging the at least one guide.
21. The ablation device of any of clauses 16 to 20, further comprising at least one electrode formed on the electrode platform.
22. The ablation device of any of clauses 16 to 21, further comprising a drive catheter extending proximally from the electrode platform.
23. The ablation device of clause 22, wherein the electrode platform is pivotable relative to the drive catheter.
24. The ablation device of any of clauses 16 to 23, wherein the angle relative to the central axis of the body is between 30 degrees and 60 degrees.
25. The ablation device of any of clauses 16 to 23, wherein the angle relative to the central axis of the body is less than 30 degrees.
26. The ablation device of any of clauses 16 to 25, wherein the at least one guide comprises a proximal portion parallel to the central axis of the body.
27. The ablation device of any of clauses 16 to 26, wherein the at least one guide comprises a distal portion angled relative to the central axis of the body.
28. The ablation device of any of clauses 16 to 27, wherein the at least one guide extends along at least a portion of the cover portion within the recess.
29. The ablation device of any of clauses 16 to 28, wherein the body comprises an angled portion formed at an angle relative to the central axis of the body.
30. The ablation device of any of clauses 16 to 29, wherein the at least one guide extends along the angled portion.
31. The ablation device of any of clauses 16 to 30, wherein the at least one guide includes a first portion extending at a first angle relative to the central axis of the body and a second portion extending at a second angle relative to the central axis of the body, the second angle being different than the first angle.
32. The ablation device of any of clauses 16 to 31, wherein the at least one guide is a channel.
33. The ablation device of any of clauses 16 to 31, wherein the at least one guide is a rail.
34. The ablation device of any of clauses 16 to 33, wherein the at least one guide comprises a first guide and a second guide, the first guide and the second guide being parallel.
35. The ablation device of any of clauses 16 to 34, wherein the body is tubular.
36. The ablation device of clause 16 according to any one or more of clauses 17 to 35.
37. A user interface of an ablation device, the user interface comprising:
   a slot having a proximal end and a distal end;
   a trigger disposed in the slot, the trigger at least partially extending through the slot, and
   being movable between the proximal end and the distal end; and,
   a drive catheter operatively coupled to the trigger, the drive catheter extending distally from the user interface and operatively coupled to an electrode platform;
   wherein the slot has a first portion and a second portion distal of the first portion;
   wherein when the trigger is in the first portion, the electrode platform is in a first orientation; and,
   wherein when the trigger is in the second portion, the electrode platform is in a second orientation, the second orientation being angled relative to the first orientation.
38. The user interface of clause 37, wherein the first portion and the second portion are separated by a resistance mechanism configured to resist the movement of the trigger from the first portion to the second portion.
39. The user interface of clause 37 or 38, further comprising a third portion proximal the first portion., wherein the third portion and the first portion are separated by a resistance mechanism configured to resist the movement of the trigger from the third portion to the first portion.
40. The user interface of clause 38 to according to any one or more of clauses 38 to 39.
41. The ablation cap of clause 15 or the ablation device of clause 16, further comprising a user interface according to any one or more of claims 37 to 39.
42. An ablation device system comprising:
   an endoscope comprising:
      an imaging device for capturing images at a distal end of the endoscope;
      one or more lumens extending through at least a portion of the endoscope between a proximal end of the endoscope and the distal end; and,
   an ablation device according to any one or more of clauses 16 to 35.
43. The ablation device system of clause 42, further comprising a user interface according to any one or more of clauses 37 to 39.

Reference throughout this specification to "one embodiment" or "an embodiment" can mean that a particular feature, structure, or characteristic described in connection with a particular embodiment can be included in at least one embodiment of claimed subject matter. Thus, appearances of the phrase "in one embodiment" or "an embodiment" in various places throughout this specification are not necessarily intended to refer to the same embodiment or to any one particular embodiment described. Furthermore, it is to be understood that particular features, structures, or characteristics described can be combined in various ways in one or more embodiments. In general, of course, these and other issues can vary with the particular context of usage. Therefore, the particular context of the description or the usage of these terms can provide helpful guidance regarding inferences to be drawn for that context.

## Claims

1. An ablation cap comprising:
a body having a lumen for receiving a distal end of an endoscope, the body having a central axis extending therethrough; and,
at least one guide for receiving at least one lateral extension of an electrode platform, wherein at least a portion of the at least one guide extends at an angle relative to the central axis of the body.

2. The ablation cap of claim 1, wherein the angle relative to the central axis of the body is one of: (a) between 30 degrees and 60 degrees, and (b) less than 30 degrees.

3. The ablation cap of claim 1 or 2, wherein the at least one guide comprises at least one of:
(a) a proximal portion parallel to the central axis of the body; and
(b) a distal portion angled relative to the central axis of the body.

4. The ablation cap of any preceding claim, further comprising a cover portion extending from a side of the body, the cover portion defining a recess between the cover portion and the body, for example wherein the at least one guide extends along at least a portion of the cover portion within the recess.

5. The ablation cap of any preceding claim, wherein the body comprises an angled portion formed at an angle relative to the central axis of the body, for example wherein the at least one guide extends along the angled portion.

6. The ablation cap of any preceding claim, wherein the at least one guide includes a first portion extending at a first angle relative to the central axis of the body and a second portion extending at a second angle relative to the central axis of the body, the second angle being different than the first angle.

7. The ablation cap of any preceding claim, wherein the at least one guide is one of: (a) a channel and (b) a rail.

8. The ablation cap of any preceding claim, wherein the at least one guide comprises a first guide and a second guide, the first guide and the second guide being parallel.

9. The ablation cap of any preceding claim, wherein the body is tubular.

10. The ablation cap according to any of the preceding claims, further comprising the electrode platform having the least one lateral extension, the electrode platform movable between a first position where the electrode platform is at a first angle relative to the central axis of the body, and a second position where the electrode platform is at a second angle relative to the central axis of the body, the second angle being different than the first angle

11. An ablation device comprising the ablation cap of any preceding claim and further comprising:
a cover portion extending from a side of the body, the cover portion defining a recess between the cover portion and the body; and
an electrode platform having said at least one lateral extension, the electrode platform movable between a covered position, where the electrode platform is covered by the cover portion, and an exposed position, where the electrode platform is not covered by the cover portion.

12. The ablation device of claim 11, wherein the at least one lateral extension of the electrode platform is slidable within the at least one guide, and/or wherein the at least one lateral extension comprises one of (a) a hook for engaging the at least one guide; (b) a wheel for engaging the at least one guide, and (c) a mechanical bearing for engaging the at least one guide.

13. The ablation device of claim 11 or 12, further comprising at least one electrode formed on the electrode platform.

14. The ablation device of any of claims 11 to 13, further comprising a drive catheter extending proximally from the electrode platform, for example wherein the electrode platform is pivotable relative to the drive catheter.

15. An ablation device system comprising:
an endoscope comprising:
an imaging device for capturing images at a distal end of the endoscope;
one or more lumens extending through at least a portion of the endoscope between a proximal end of the endoscope and the distal end; and,
an ablation device according to any one or more of claims 11 to 14.
